# EUROPEAN PATENT SPECIFICATION

(11) **EP 0 698 111 B1**
(45) Date of publication and mention of the grant of the patent: **19.02.2003**
(21) Application number: 94917312.4
(22) Date of filing: 06.05.1994
(51) Int. Cl.: C12P 17/06

(54) **PROCESS FOR SYNTHESIS OF HMG-CoA REDUCTASE INHIBITORS**
SYNTHESEVERFAHREN FÜR INHIBITOREN DER HMG-CoA-REDUKTASE
PROCEDE DE SYNTHESE D'INHIBITEURS DE LA HMG-CoA REDUCTASE

(30) Priority: 11.05.1993 US 60847
(43) Date of publication of application: 28.02.1996
(73) Proprietor: Merck & Co., Inc., Rahway New Jersey 07065-0900 (US); THE UNIVERSITY OF VIRGINIA ALUMNI PATENTS FOUNDATION, Charlottesville, VA 22903 (US)
(72) Inventor: CARTA, Giorgio, Charlottesville, VA 22901 (US); CONDER, Michael J., Harrisonburg, VA 22801 (US); GAINER, John Lloyd, Charlottesville, VA 22901 (US); STIEBERG, Robert W., Harrisonburg, VA 22801 (US); VINCI, Victor A., Charlottesville, VA 22901 (US); WEBER, Timothy Wallace, Grampian, PA 16838 (US)
(74) Representative: Thompson, John Dr.
(86) International application number: PCT/US94/05019
(87) International publication number: WO 94/026920

(56) References cited:
- GB-A- 2 255 974
- JP-A- 60 176 595
- US-A- 5 223 415
- PATEL R. N. ET AL.: "Enantioselective Enzymatic Acetylation." APPL. MICROBIOL. BIOTECHNOL., vol. 38, no. 1, 1992, pages 56-60, XP002069330
- Biochimica et Biophysica Acta, Volume 575, issued 1979, OKUMURA et al., "Synthesis of Various Kinds of Esters by Four Microbial Lipases", pages 156-165, see Abstract.
- Tetrahedron, Volume 42, Number 13, issued 1986, J.B. JONES, "Enzymes in Organic Synthesis", pages 3351-3403, see Fig. 1 on page 3353.
- Journal of the American Chemical Society, Volume 110, issued 1988, WANG et al., "Lipase-Catalyzed Irreversible Transesterifications Using Enol Esters as Acylating Reagents: Preparative Enantio- and Regioselective Syntheses of Alcohols, Glycerol Derivatives, Sugars, and Organometallics", pages 7200-7205, see page 7201.
- Tetrahedron Letters, Volume 27, Number 1, issued 1986, LANGRAND et al., "Lipase Catalyzed Reactions and Strategy for Alcohol Resolution", pages 29-32, see page 29.
- The Journal of Antibiotics, Volume XXXIX, Number 11, issued November 1986, KOMAGATA et al., "Microbial Conversion of Compactin (ML-236B) to ML-236A", pages 1574-1577, see Abstract.
- Biochimica et Biophysica Acta, Volume 1042, issued 1990, HILLS et al., "Lipase from Brassica Napus L. Discriminates against Cis-4 and Cis-6 Unsaturated Fatty Acids and Secondary and Tertiary Alcohols", pages 237-240, see Abstract.

## Description

### BACKGROUND OF THE INVENTION

HMG-CoA reductase inhibitors containing a polyhydronaphthyl group as the hydrophobic moiety may be prepared by a multistep chemical synthesis from the diol lactone which has previously been blocked at the lactone hydroxyl with a silyloxy protecting group, or in some cases, these compounds may be prepared by chemical modification of the natural products mevastatin and lovastatin. Mevastatin and lovastatin are prepared by biosynthetic fermentation processes.

The present invention provides a highly advantageous, one-step, efficient process which may be employed to prepare HMG-CoA reductase inhibitors, such as lovastatin, from the available diol lactone.

GB-A-2 255 974, JP-A-60176595 and *J. Antibiot.* **39**(11), 1574-7 (1986), disclose lipase-catalysed deacylation reactions. In *Appl. Microbiol. Biotechnol.* 38(1), 55-60 (1992), *J. Am. Chem. Soc*. **110**(21), 7200-5 (1988) and *Tetrahedron* **42**(13), 3351-3403 (1986), it is disclosed that such deacylations may be reversible. *Tetrahedron Lett.* **27**(1), 29-32 (1986) discloses the lipase-catalysed acylation of a cycloalkyl mono-ol.

### DESCRIPTION OF THE INVENTION

This invention relates to an esterification process for forming HMG-CoA reductase inhibitors of formula (I): 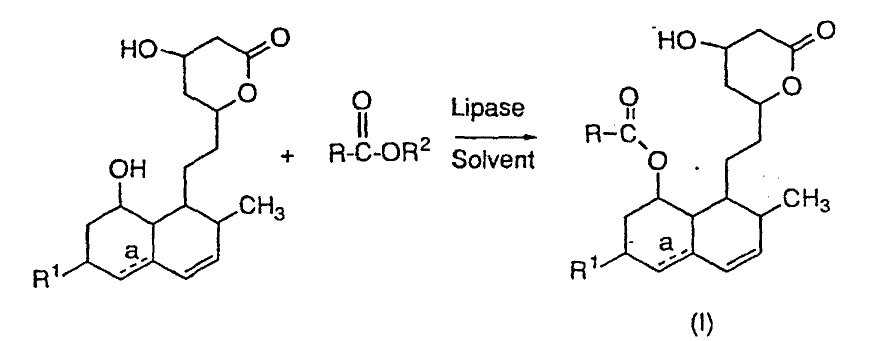 wherein
R is C₁₋₅alkyl;
R¹ is H, CH₃, or OH;
R² is H or C₁₋₅alkyl, and
a indicates the optional presence of a double bond.

More specifically, the process employs an immobilized lipase enzyme in a nonaqueous organic solvent. The source of the lipase enzyme may be selected from the group listed in Table I; the organisms listed therein have been shown to produce enzymes capable of deacylating lovastatin to triol acid (EP 486,153, published May 20, 1992). This list is not intended to be exhaustive and should be understood to include other organisms that produce enzymes which catalyze the side-chain removal of lovastatin and compactin. The process of the present invention preferably employs an immobilized enzyme (lipase or esterase); a specific example of a suitable immobilization procedure for use with a commercially available enzyme is given in Example 1 and further discussed below. Other methods for obtaining immobilized enzyme include growing up the producing organism in a suitable nutrient medium, harvesting the microbial cells and lyophilizing them. This illustrates the simplest form of immobilization; however, such a method has the potential drawbacks of the present enzyme which has not been purified in any way, thereby making the specific activity of the preparation lower than when a more purified preparation is employed, and the presence of the microbial cell wall which may act as a permeability barrier to the reactants (substrate), and products. More preferable methods utilize enzymes which have been released from microbial cells (through mechanical or enzymatic breakage) and purified to some extent. The means by which proteins (enzymes) can be purified from complex biological mixtures are well-known to those skilled in the art and include centrifugation, ultrafiltration, salting out (with e.g. ammonium sulfate), various types of column chromatography (ion-exchange, hydrophobic interaction, gel filtration and affinity) and electrophoresis. The purification of the desired enzyme can be conveniently monitored throughout the purification process by assaying putative enzyme-containing fractions from the various purification steps for the ability to hydrolyze the side chain from lovastatin acid (which can be carried out in aqueous solution and monitored via HPLC analysis). The choice of immobilization support useful for the attachment of the enzyme is not confined to nylon (as used in the Example given) but may be any other support used by the skilled artisan for enzyme immobilization. Examples include polyacrylamide, agarose and other commercially available, activated support resins such as Emphase™ (3M Corp.) and Eupergit® C (Rohm Pharma). It is possible that some organisms may produce extracellular forms of lipase or esterase; purification of such enzymes can also be accomplished using the methods discussed above, however, the step of releasing the enzyme from the producing cells would not be necessary in this case.

A particular example of such a lipase enzyme is lipase type VII derived from *Candida cylindracea* C (from the Sigma Chemical Co). This enzyme is commercially available. The lipase enzyme is conveniently immobilized on nylon 6 pellets employing the procedure of Example 1. The nonaqueous organic solvent may be selected from any organic solvent capable of dissolving the stated diol lactone without deactivating the enzyme. Particular examples of such solvents include mixtures of a chlorinated hydrocarbon such as chloroform or methylene chloride and a hydrocarbon such as hexane or toluene. Specifically illustrating such a mixture is a 50/50 mixture by volume of chloroform and hexane. The reaction may take place between stoichiometric proportions of acid or ester and diol lactone or excesses of the acid or ester may be used. The temperature employed may be 15-40°C with a preferred range being 20-37°C.

Compounds of formula (I) which may be prepared include those wherein R is ethyl, n-propyl, 2-butyl or 2-methyl-2-butyl. The starting lactones have been previously reported in the literature and their preparation is known to those skilled in the art. US Patent 5,250,435 provides an alternate source of diol lactone. Alternatively, one of the enzymes described in Japanese Patent Publication JP60-176595 can be used to remove the side-chain of HMG-CoA reductase inhibitors to produce acid starting material. US Patent 4,293,496 discloses side-chain removal employing base hydrolysis; US Patent 5,223,415 discloses diol lactone formation using the enzyme *Clonostachys compactiuscula.* The current process to produce compactin (ML-236B) and pravastatin (PRAVACHOL) is disclosed in US Patent 4,346,227. Briefly, this process involves fermentation of *Penicillium citrinum* to produce compactin, followed by hydroxylation of compactin at C6 using microorganisms of the genera Mucor, Rhizopus, Zygorynchus, Circinella, Actinomucor, Gongronella, Phycomyces, Martierella, Pycnoporus, Rhizoctonia, Absidia, Cunninghamella, Syncephalastrum and Streptomyces, or cell free enzyme-containing extracts from these microorganisms. One method to prepare compounds wherein R¹ is hydroxyl is to produce mutants of microorganisms producing compactin, such as *Penicillium citrinum* which produce compactin diol, and to harvest the compactin diol. Then enzymatic esterification of the 8-hydroxyl group of compactin is accomplished according to the process of the present invention, followed by hydroxylation as described above using microorganisms or extracts thereof.

The starting carboxylic acids and their corresponding esters are all commercially available. The lipase enzymes are either available from commercial sources or the microbes which make the enzyme have been deposited with the ATCC and are available from that source.

**TABLE 1**

| strain name | ATCC No. |
|---|---|
| *Mortierella isabellina* | 42013 |
| *Mucor circinelloides* | 1207a |
| *Fusarium solani* | 12826 |
| *Dichotomomyces cejpii* | 22149 |
| *Dichotomomyces cejpii* | 42284 |
| *Diheterospora chlamydosporia* | 16449 |
| *Diheterospora chlamydosporia* | 18056 |
| *Diheterospora chlamydosporia* | 20537 |
| *Emericella unguis* | 10073 |
| *Emericella unguis* | 12063 |
| *Emericella unguis* | 13431 |
| *Emericella unguis* | 16812 |
| *Humicola fuscoatra* | 12774 |
| *Humicola fuscoatra* | 52037 |
| *Humicola fuscoatra* | 62175 |
| *Mortierella isabellina* | 36670 |
| *Mortierella isabellina* | 38063 |
| *Mortierella isabellina* | 44853 |
| *Neocosmospora africana* | 24342 |
| *Xylogone sphaerospora* | 42027 |
| *Penicillium chrysogenum* | 10002 |
| *Aspergillus clavatus* | 1007 |
| *Gilmaniella humicola* | 16013 |
| *Mucor bainieri* | 42642 |
| *Chaetomium cochliodes* | 10195 |
| *Clonostachys compactiuscula* | 38009 |
| | 74178 |
| *Candida cylindracea C* (Sigma Chemical Co.) | |
| *Candida cylindracea* | 14830 |

### EXAMPLE 1

### Procedure For Enzyme Immobilization

Approximately 10 g of Nylon 6 pellets, previously frozen and ground to between 425 and 1180 µm, was weighed out.

A pH 7 phosphate buffer was prepared by adding Na₂HPO₄•7H₂O to deionized water to obtain pH 9.0 ± 0.5 followed by addition of 1N HCl to bring the pH to 7.0.

A 2.5% glutaraldehyde solution was prepared by adding to 10 mL of a 25% solution enough pH 7 phosphate buffer to make 100 mL.

6N HCl was added to the nylon pellets in a beaker and stirred constantly for 10-15 seconds. The acid was decanted and the pellets rinsed with deionized water until clear; a spatula was used to break up lumps of hydrolyzed nylon.

The nylon was filtered on a buchner funnel to achieve dryness.

The dried nylon clumps were broken up in a mortar and pestle and then transfered to a flask with 100 mL of the 2.5% glutaraldehyde solution. The mixture was stirred for at least 1 hour.

The enzyme attachment solution was prepared by mixing 4.8 g of lipase Type VII derived from *Candida cylindracea* (Sigma Chemical Co.) in 30 mL of the pH 7 phosphate buffer in a 50 mL culture tube. The mixture was shaken to get as much lipase into solution as possible.

The previously stirred nylon glutaraldehyde solution was decanted and the nylon washed with 100 mL of deionized water and then again with 500 mL of deionized water. The nylon was filtered and dried.

A portion of the enzyme attachment solution (0.5 mL) was drawn for protein analysis, and the nylon pellets were added to the enzyme attachment solution. The bottle was rolled for 18-24 hours and a second aliquot was removed for protein analysis.

The attachment solution was decanted, and the nylon washed with deionized water. The nylon was spread out and allowed to dry overnight.

### EXAMPLE 2

### Isolation and Extraction of Diol Lactone

Wherein R¹ is methyl.

### a. Triol Workup

The triol acid corresponding to the diol lactone was prepared according to the procedure in EPO publication 517,413 published December 19, 1992. Ammonium sulfate was added to the fermentation broth in an amount sufficient to bring the concentration to 0.25 M. Isopropyl acetate was added in volume equal to 0.8 times the fermentation broth followed by the addition of isopropyl alcohol in equal volume to 0.05 times the isopropyl acetate volume. The broth was adjusted to pH 4.0 with 2N sulfuric acid and then agitated for 30 minutes, centrifuged, and the upper isopropyl acetate layer recovered.

### b. Carbonate Extraction

Dilute carbonate solution (25 g/L Na₂CO₃) was added to the isopropyl acetate solution in volume equal to 0.4 x the isopropyl acetate. The resulting solution was agitated for 30 minutes, centrifuged and the upper isopropyl acetate and lower carbonate aqueous layer separated.

### c. Second Isopropyl Acetate Extraction

An equal volume of isopropyl acetate was added to the carbonate solution. The pH was adjusted to 4.0 with 85% H₃PO₄ and the solution agitated for 30 minutes centrifuged and the upper isopropyl acetate layer recovered.

### d. Lactonization

70% methanesulfonic acid was added to the recovered isopropyl acetate solutions to achieve a final concentration of 10 mM. The resulting mixture was evaporated in vacuo at 33-34°C to 20% of the original volume.

### e. Dilute Carbonate Wash

The lactonized solution from step (d) was added to a separatory funnel and cold, dilute carbonate was added in an equal volume to isopropyl acetate. The mixture was agitated for 1-2 minutes over ice and the upper isopropyl acetate layer was recovered.

### f. Crystallization

The covered solution from step (e) was stored in a freezer while crystals were formed. The crystals were filtered and the excess isopropyl acetate was collected. The crystals were washed with cold toluene/isopropyl acetate (9:1) until the wash was clear. The crystals were vacuum dried at room temperature.

### EXAMPLE 3

### Enzymatic Acylation of Lovastatin Diol Lactone to form Lovastatin

A reaction mixture containing the following components was prepared.

| | |
|---|---|
| Lovastatin Diol Lactone | 50.5 mg |
| 2-Methylbutyric acid | 452 µL |
| Chloroform | 5.0 mL |
| hexane | 5.0 mL |
| Nylon Immobilized Enzyme (prepared in Example 1) | 1.0 g |

The 10 mL reaction mixture was incubated in a screw-capped test tube and placed on a rotator equipped with a disc containing test tube holders. The reactions were carried out at 25°C or 37°C while the tubes rotated at approximately 60 rpm for 2 to 24 hours with sample taken at regular intervals. Lovastatin was detected by HPLC.
Lovastatin separation was on a C₁₈ reverse phase column using a mobile phase of 50% acetonitrile and 50% H₃PO₄ in H₂O at a flow rate of 3 ml/min. Detection was at 238 nm.

### EXAMPLE 4

### Enzymatic Acylation of Compactin Diol Lactone

### a. Preparation of diol lactone wherein R¹ is H (Compactin Diol Lactone)

The starting diol lactone wherein R¹ is H is prepared by hydrolyzing compactin.

A mixture of 200 mg compactin in 5 mL of aqueous 1N LiOH solution is shaken for 12 h at 135°C in a 30 mL stainless steel pressure vessel. The cooled reaction mixture is acidified with 1M H₃PO₄ and extracted with ethyl acetate. The ethyl acetate solution is dried over magnesium sulfate, filtered, and concentrated in vacuo. The residue is dissolved in 20 mL toluene heated to reflux for 4 h in a Dean-Stark apparatus to effect lactonization. Evaporation of the toluene gives the diol lactone wherin R¹ is H (compactin diol lactone).

### b. Enzymatic Acylation of the Compactin Diol Lactone

A reaction mixture containing the following components is prepared:

| | |
|---|---|
| Compactin Diol Lactone | 50 mg |
| 2-Methylbutyric acid | 450 µL |
| Chloroform | 5 mL |
| hexane | 5 mL |
| Nylon Immobilized Enzyme (prepared in Example 1) | 1.0 g |

The 10 mL reaction mixture is incubated according to the procedures of Example 3. Compactin is detected, separated and quantitated by reverse phase HPLC.

### EXAMPLE 5

### Enzymatic Acylation of Pravastatin

### Diol Lactone

### a. Preparation of Diol Lactone wherein R¹ is OH

Starting with Pravastatin, (3R,5R) 3,5-dihydroxy-7-[(1S, 2S, 6S, 8S, 8aR)-6-hydroxy-2-methyl-8-[(S)-2-methyl-butyryloxy]-1,2,6,7,8,8a-hexahydro-1-naphthyl]heptanoate and following the procedures of Example 4, step a, pravastatin diol lactone is formed (wherein R¹ is OH).

### b. Enzymatic Acylation of Pravastatin Diol Lactone

A reaction mixture containing the following components is prepared:

| | |
|---|---|
| Pravastatin Diol Lactone (R¹ is OH) | 50 mg |
| Propionic acid | 425 µL |
| Chloroform | 5 mL |
| Hexane | 5 mL |
| Nylon Immobilized Enzyme (prepared in Example 1) | 1.0 g |

The 10 mL reaction mixture is incubated according to the procedures of Example 3. The product is detected and purified by reverse phase HPLC.

### EXAMPLE 6

### Kinetic Data for Lovastatin Production at 37°C

As described in Example 3 above the lovastatin diol lactone starting material wherein R¹ is CH₃ was dissolved in 50% chloroform, 50% hexane (v:v) and 2-methyl butyric acid was added as well as nylon immobilized lipase derived from *Candida cylindracea* (prepared according to the procedures of Example 1).

| Time (hour) | Diol Conc. (g/L) | Lovastatin Conc. (g/L) |
|---|---|---|
| 0 | 4.12 | 0 |
| 1 | 2.87 | 0 |
| 2 | 1.76 | 0 |
| 3 | 3.02 | 0.04 |
| 24 | 3.61 | 0.74 |

Taken at 3 hours, the data above give a production rate of 3.1 x 10⁻⁵ mol/[hr -g lipase]. At 24 hours, however, the rate is calculated to be 7.1 x 10⁻⁵ mol/[hr -g lipase].

### EXAMPLES 7-12

### Enzymatic Acylation Lovastatin Diol Lactone to Produce Various Esters

Reactions were run with lovastatin diol lactone at room temperature (20-25°C) employing lipase enzyme from *Candida cylindracea* purchased from Sigma Chemical Company (Type VII) and immobilized according to the procedures in Example 1 in the specification. The reactions were carried out in 50% chloroform, 50% hexane (v:v).

| Examples | Acid Substrate | Esterification Rate (mol/ [h -g lipase]) |
|---|---|---|
| 7 | Propionic acid | 7.3 x 10⁻⁵ |
| 8 | n-Butyric acid | 2.1 x 10⁻⁴ |
| 9 | 2-Methyl butyric acid | 3.2 x 10⁻⁵ (averaged value) |
| 10 | 3-Methyl butyric acid | 2.2 x 10⁻⁶ |
| 11 | 2,2-Dimethyl butyric acid | 0 |
| 12 | (E)-2-Methyl-2-butenoic acid (Tiglic acid) | 0 |

## Claims

1. A process for the formation of a compound of formula (I): 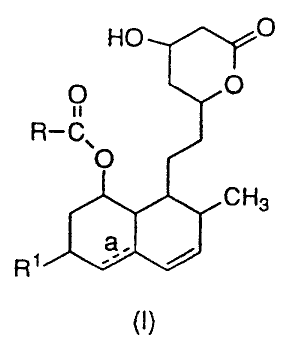 which comprises treating a diol lactone: 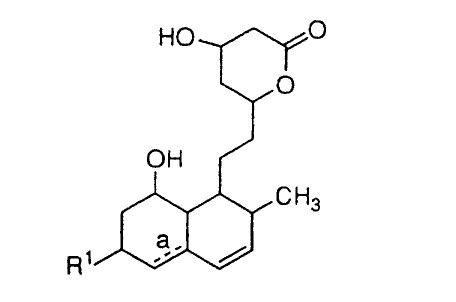 with R-COOR² in a nonaqueous organic solvent containing
an immobilised lipase enzyme,
wherein:
R is C₁₋₅ alkyl;
R¹ is H, CH₃ or OH;
R² is H or C₁₋₅alkyl, and
a indicates the optional presence of a double bond,
and recovering the compound of structural formula (I).

2. The process of claim 1 wherein the lipase enzyme is derived from a microorganism selected from:
(1) *Mortierella isabellina* ATCC 42013,
(2) *Mucor circinelloides* ATCC 1207a,
(3) Fusarium solani ATCC 12826,
(4) *Dichotomomyces cejpii* ATCC 22149,
(5) *Dichotomomyces cejpii* ATCC 42284,
(6) *Diheterospora chlamydosporia* ATCC 16449,
(7) *Diheterospora chlamydosporia* ATCC 18056,
(8) *Diheterospora chlamydosporia* ATCC 20537,
(9) *Emericella unguis* ATCC 10073,
(10) *Emericella unguis* ATCC 12063,
(11) *Emericella unguis* ATCC 13431,
(12) *Emericella unguis* ATCC 16812,
(13) *Humicola fuscoatra* ATCC 12774,
(14) *Humicola fuscoatra* ATCC 52037,
(15) *Humicola fuscoatra* ATCC 62175,
(16) *Mortierella isabellina* ATCC 36670,
(17) *Mortierella isabellina* ATCC 38063,
(18) *Mortierella isabellina* ATCC 44853,
(19) *Neocosmospora africana* ATCC 24342,
(20) *Xylogone sphaerospora* ATCC 42027,
(21) *Penicillium chrysogenum* ATCC 10002,
(22) *Aspergillus clavatus* ATCC 1007,
(23) *Gilmaniella humicola* ATCC 16013,
(24) *Mucor bainieri* ATCC 42642,
(25) *Chaetomium cochliodes* ATCC 10195,
(26) *Clonostachys compactiuscula* ATCC 38009 and ATCC 74178,
(27) *Candida cylindracea* ATCC 14830; and
(28) *Candida cylindracea C.*

3. The process of claim 2 wherein the lipase enzyme is lipase type VII derived from *Candida cylindracea* C.

4. The process of Claim 2 wherein R is 2-butyl; R¹ is CH₃; and R² is H.

5. The process of Claim 2 wherein a is a double bond.

6. The process of Claim 4 wherein a is a single bond.

7. The process of Claim 4 wherein the organic solvent is a mixture of a chlorinated hydrocarbon and a hydrocarbon.

8. The process of Claim 5 wherein the lipase enzyme is derived from *Candida cylindracea* ATCC 14830 or *Candida cylindracea* C.

9. The process of Claim 6 wherein the organic solvent is a 1:1 mixture by volume of chloroform and hexane.

10. The process of Claim 2 wherein R is 2-methyl-2-butyl, R¹ is CH₃ and R² is H.

11. The process of Claim 10 wherein a is a double bond.

12. The process of Claim 2 wherein R is 2-butyl, R¹ is OH, and R² is H.

13. The process of Claim 12 wherein a is a double bond.

14. The process of claim 3 wherein the organic solvent is a mixture of a chlorinated hydrocarbon and a hydrocarbon.

15. The process of claim 14 wherein the organic solvent is a 1:1 mixture by volume of chloroform and hexane.

16. The process of claim 2 wherein:
(i) the lipase enzyme is derived from a microorganism selected from those numbered from (1) to (27);
(ii) R² is H, and R and R¹ are such that
(a) R is 2-butyl and R¹ is CH₃,
(b) R is 2-methyl-2-butyl and R¹ is CH₃, or
(c) R is 2-butyl and R¹ is OH; and
(iii) the organic solvent is a mixture of a chlorinated hydrocarbon and a hydrocarbon.

17. The process of claim 16 wherein the organic solvent is a 1:1 mixture by volume of chloroform and hexane.

18. The process of claim 16 or claim 17 wherein R and R¹ are such that R is 2-methyl-2-butyl and R¹ is CH₃, or R is 2-butyl and R¹ is OH;
and a is a double bond.

19. The process of claim 15 wherein R is 2-butyl, R¹ is CH₃, R² is H and a is a double bond.

## Patentansprüche

1. Ein Verfahren zur Bildung einer Verbindung der Formel (I): 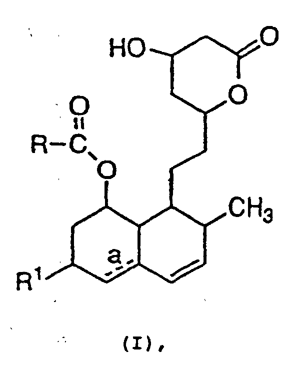 das die Behandlung eines Diollactons: 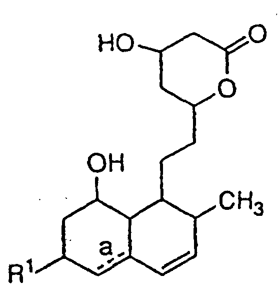 mit R-COOR² in einem nichtwäßrigen organischen Lösungsmittel, das ein immobilisiertes Lipaseenzym enthält,
wobei:
R C₁₋₅-Alkyl ist,
R¹ H, CH₃ oder OH ist,
R² H oder C₁₋₅-Alkyl ist und
a die optionale Anwesenheit einer Doppelbindung bedeutet,
und die Gewinnung der Verbindung der Strukturformel (I) umfaßt.

2. Das Verfahren nach Anspruch 1, wobei das Lipaseenzym von einem Mikroorganismus abgeleitet ist, ausgewählt aus:
(1) *Mortierella isabellina* ATCC 42013,
(2) *Mucor circinelloides* ATCC 1207a,
(3) *Fusarium solani* ATCC 12826,
(4) *Dichotomomyces cejpii* ATCC 22149,
(5) *Dichotomomyces cejpii* ATCC 42284,
(6) *Diheterospora chlamydosporia* ATCC 16449,
(7) *Diheterospora chlamydosporia* ATCC 18056,
(8) *Diheterospora chlamydosporia* ATCC 20537,
(9) *Emericella unguis* ATCC 10073,
(10) *Emericella unguis* ATCC 12063,
(11) *Emericella unguis* ATCC 13431,
(12) *Emericella unguis* ATCC 16812,
(13) *Humicola fuscoatra* ATCC 12774,
(14) *Humicola fuscoatra* ATCC 52037,
(15) *Humicola fuscoatra* ATCC 62175,
(16) *Mortierella isabellina* ATCC 36670,
(17) *Mortierella isabellina* ATCC 38063,
(18) *Mortierella isabellina* ATCC 44853,
(19) *Neocosmospora africana* ATCC 24342,
(20) *Xylogone sphaerospora* ATCC 42027,
(21) *Penicillium chrysogenum* ATCC 10002,
(22) *Aspergillus clavatus* ATCC 1007,
(23) *Gilmaniella humicola* ATCC 16013.
(24) *Mucor bainieri* ATCC 42642,
(25) *Chaetomium cochliodes* ATCC 10195,
(26) *Clonostachys compactiuscula* ATCC 38009 und ATCC 74178,
(27) *Candida cylindracea* ATCC 14830 und
(28) *Candida cylindracea* C.

3. Das Verfahren nach Anspruch 2, wobei das Lipaseenzym Lipasetyp VII ist, der von *Candida cylindracea* C. abgeleitet ist.

4. Das Verfahren nach Anspruch 2, wobei R 2-Butyl ist, R¹ CH₃ ist und R² H ist.

5. Das Verfahren nach Anspruch 2, wobei a eine Doppelbindung ist.

6. Das Verfahren nach Anspruch 4, wobei a eine Einfachbindung ist.

7. Das Verfahren nach Anspruch 4, wobei das organische Lösungsmittel eine Mischung aus einem chlorierten Kohlenwasserstoff und einem Kohlenwasserstoff ist.

8. Das Verfahren nach Anspruch 5, wobei das Lipaseenzym von *Candida cylindracea* ATCC 14830 oder *Candida cylindracea* C. abgeleitet ist.

9. Das Verfahren nach Anspruch 6, wobei das organische Losungsmittel eine 1:1-Mischung, bezogen auf das Volumen, aus Chloroform und Hexan ist.

10. Das Verfahren nach Anspruch 2, wobei R 2-Methyl-2-butyl ist, R¹ CH₃ ist und R² H ist.

11. Das Verfahren nach Anspruch 10, wobei a eine Doppelbindung ist.

12. Das Verfahren nach Anspruch 2, wobei R 2-Butyl ist, R¹ OH ist und R² H ist.

13. Das Verfahren nach Anspruch 12, wobei a eine Doppelbindung ist.

14. Das Verfahren nach Anspruch 3, wobei das organische Lösungsmittel eine Mischung aus einem chlorierten Kohlenwasserstoff und einem Kohlenwasserstoff ist.

15. Das Verfahren nach Anspruch 14, wobei das organische Lösungsmittel eine 1:1-Mischung, bezogen auf das Volumen, aus Chloroform und Hexan ist.

16. Das Verfahren nach Anspruch 2, wobei:
(i) das Lipaseenzym von einem Mikroorganismus abgeleitet ist, ausgewählt aus denen mit den Nummern (1) bis (27),
(ii) R² H ist und R und R¹ derart sind, daß
(a) R 2-Butyl ist und R¹ CH₃ ist,
(b) R 2-Methyl-2-butyl ist und R¹ CH₃ ist oder
(c) R 2-Butyl ist und R¹ OH ist, und
(iii) das organische Lösungsmittel eine Mischung aus einem chlorierten Kohlenwasserstoff und einem Kohlenwasserstoff ist.

17. Das Verfahren nach Anspruch 16, wobei das organische Lösungsmittel eine 1:1-Mischung, bezogen auf das Volumen, aus Chloroform und Hexan ist.

18. Das Verfahren nach Anspruch 16 oder Anspruch 17, wobei R und R¹ derart sind, daß R 2-Methyl-2-butyl ist und R¹ CH₃ ist, oder daß R 2-Butyl ist und R¹ OH ist,
und a eine Doppelbindung ist.

19. Das Verfahren nach Anspruch 15, wobei R 2-Butyl ist, R¹ CH₃ ist, R² H ist und a eine Doppelbindung ist.

## Revendications

1. Procédé de formation d'un composé de formule (I) : 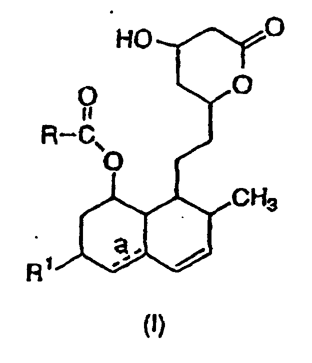 qui comprend le traitement d'une diol-lactone : 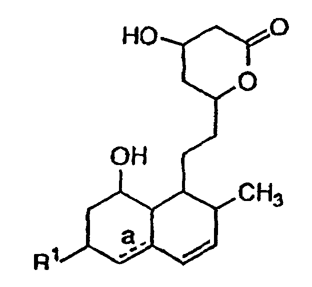 avec R-COOR² dans un solvant organique non aqueux contenant une enzyme lipase immobilisée,
dans lequel :
R est un groupe alkyle en C₁₋₅;
R¹ est H, CH₃ ou OH;
R² est H ou un groupe alkyle en C₁₋₅; et
a indique la présence éventuelle dune double liaison,
et la récupération du composé de formule développée (I).

2. Procédé selon la revendication 1 dans lequel l'enzyme lipase provient d'un microorganisme choisi parmi :
(1) *Mortierella isabellina* ATCC 42013,
(2) *Mucor circinelloides* ATCC 1207a,
(3) *Fusarium solani* ATCC 12826,
(4) *Dichotomomyces cejpii* ATCC 22149,
(5) *Dichotomomyces cejpii* ATCC 42284,
(6) *Diheterospora chlamydosporia* ATCC 16449,
(7) *Diheterospora chlamydosporia* ATCC 18056,
(8) *Diheterospora chlamydosporia* ATCC 20537,
(9) *Emericella unguis* ATCC 10073,
(10) *Emericella unguis* ATCC 12063,
(11) *Emericella unguis* ATCC 13431,
(12) *Emericella unguis* ATCC 16812,
(13) *Humicola fuscoatra* ATCC 12774,
(14) *Humicola fuscoatra* ATCC 52037,
(15) *Humicola fuscoatra* ATCC 62175,
(16) *Mortierella isabellina* ATCC 36670,
(17) *Mortierella isabellina* ATCC 38063,
(18) *Mortierella isabellina* ATCC 44853,
(19) *Neocosmospora africana* ATCC 24342,
(20) *Xylogone sphaerospora* ATCC 42027,
(21) *Pénicillium chrysogenum* ATCC 10002,
(22) *Aspergillus clavatus* ATCC 1007,
(23) *Gilmaniella humicola* ATCC 16013,
(24) *Mucor bainieri* ATCC 42642,
(25) *Chaetomium cochliodes ATCC 10195,*
(26) *Clonostachys compactiuscula* ATCC 38009 et ATCC 74178,
(27) *Candida cylindracea* ATCC 14830; et
(28) *Candida cylindracea* C.

3. Procédé selon la revendication 2 dans lequel l'enzyme lipase est la lipase de type VII provenant de *Candida cylindracea* C.

4. Procédé selon la revendication 2 dans lequel R est un groupe 2-butyle; R¹ est CH₃; et R² est H.

5. Procédé selon la revendication 2 dans lequel a est une double liaison.

6. Procédé selon la revendication 4 dans lequel a est une simple liaison.

7. Procédé selon la revendication 4 dans lequel le solvant organique est un mélange d'un hydrocarbure chloré et d'un hydrocarbure.

8. Procédé selon la revendication 5 dans lequel l'enzyme lipase provient de *Candida cylindracea* ATCC 14830 ou de *Candida cylindracea* C.

9. Procédé selon la revendication 6 dans lequel le solvant organique est un mélange de chloroforme et d'hexane à 1/1 en volume.

10. Procédé selon la revendication 2 dans lequel R est un groupe 2-méthyl-2-butyle, R¹ est CH₃ et R² est H.

11. Procédé selon la revendication 10 dans lequel a est une double liaison.

12. Procédé selon la revendication 2 dans lequel R est un groupe 2-butyle, R¹ est OH et R² est H.

13. Procédé selon la revendication 12 dans lequel a est une double liaison.

14. Procédé selon la revendication 3 dans lequel le solvant organique est un mélange d'un hydrocarbure chloré et d'un hydrocarbure.

15. Procédé selon la revendication 14 dans lequel le solvant organique est un mélange de chloroforme et d'hexane à 1/1 en volume.

16. Procédé selon la revendication 2 dans lequel :
(i) l'enzyme lipase provient d'un microorganisme choisi parmi ceux énumérés de (1) à (27) ;
(ii) R² est H et R et R¹ sont tels que
(a) R est un groupe 2-butyle et R¹ est CH₃,
(b) R est un groupe 2-méthyl-2-butyle et R¹ est CH₃, ou
(c) R est un groupe 2-butyle et R¹ est OH; et
(iii) le solvant organique est un mélange d'un hydrocarbure chloré et d'un hydrocarbure.

17. Procédé selon la revendication 16 dans lequel le solvant organique est un mélange de chloroforme et d'hexane à 1/1 en volume.

18. Procédé selon la revendication 16 ou la revendication 17 dans lequel R et R¹ sont tels que R est un groupe 2-méthyl-2-butyle et R¹ est CH₃, ou R est un groupe 2-butyle et R¹ est OH;
et a est une double liaison.

19. Procédé selon la revendication 15 dans lequel R est un groupe 2-butyle, R¹ est CH₃, R² est H et a est une double liaison.
